# EUROPÄISCHE PATENTSCHRIFT

(11) **EP 1 393 707 B1**
(45) Veröffentlichungstag und Bekanntmachung des Hinweises auf die Patenterteilung: **31.05.2006**
(21) Anmeldenummer: 03014576.7
(22) Anmeldetag: 07.07.2003
(51) Int. Cl.: A61K 8/06, A61K 8/37, A61Q 5/00, A61Q 5/02, A61Q 19/00

(54) **Kosmetikum zur Reinigung und Pflege der Haut**
Cosmetic composition for cleaning and care of the skin
Conmposition cosmétique pour nettoyer et soigner la peau

(30) Priorität: 17.08.2002 DE 10237735
(43) Veröffentlichungstag der Anmeldung: 03.03.2004
(73) Patentinhaber: Beiersdorf AG, 20245 Hamburg (DE)
(72) Erfinder: Ruppert, Stephan Dr., 20259 Hamburg (DE); Counradi, Katrin, 22143 Hamburg (DE); Treu, Jens Dr., 22844 Norderstedt (DE); Rohde, Olaf, 22880 Wedel (DE); Kröpke, Rainer, 22869 Schenefeld (DE)

(56) Entgegenhaltungen:
- DE-A- 19 725 087
- US-A1- 2002 045 670

## Beschreibung

Die vorliegende Erfindung betrifft pflegende Kosmetika und/oder Dermatika zur Reinigung und Pflege der Haut

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut und der Hautanhangsgebilde

Damit die Haut und die Hautanhangsgebilde, hierzu zählen vor allem die Haare und Nägel, ihre biologischen Funktionen im vollen Umfang erfüllen können, bedürfen sie der regelmäßigen Reinigung und Pflege sowie dem Schutz vor UV-Strahlung. Die Reinigung dient dabei der Entfernung von Schmutz, Schweiß und Resten abgestorbener Körperzellen, die einen idealen Nährboden für Krankheitserreger und Parasiten aller Art bilden. Kosmetische Reinigungsprodukte werden in der Regel in Form von Gelen, Lotionen und Feststoffen (Seifenstücke, Waschsynthets) angeboten. Hautpflegeprodukte, in der Regel Crémes, Salben oder Lotionen, dienen meist der Befeuchtung und Rückfettung der Haut, denn die Aufgabe der Hautpflege ist es, den durch das tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern. Häufig sind Hautpflegeprodukten Wirkstoffe zugesetzt, welche die Haut regenerieren und beispielsweise ihre vorzeitige Alterung (z.B. das Entstehen von Fältchen, Falten) verhindern und vermindern sollen. Zum Schutz vor der schädlichen UV-Strahlung des Sonnenlichtes sind vielen kosmetischen und dermatologischen Produkten UV-Lichtschutzfilter zugesetzt

Die weitaus meisten kosmetischen und dermatologischen Zubereitungen enthalten grenzflächenaktive Verbindungen, die eine Aufnahme und Stabilisierung von Lipiden in Wasser bzw. von Wasser in Lipiden ermöglichen. Man bezeichnet solche Verbindungen meist als Tenside oder Emulgatoren. Tenside und Emulgatoren zeichnen sich durch ein ambivalentes Verhalten gegenüber Wasser und Lipiden aus: Die Moleküle enthalten mindestens je eine hydrophile und eine lipophile Gruppe, die die Anlagerung an der Grenzfläche zwischen diesen beiden Substarizktassen ermöglichen

Was unterscheidet nun waschaktive Tenside von Emulgatoren?

Tenside sorgen für eine Herabsetzung der Oberflächenspannung des Wassers, die Benetzung der Haut, die Erleichterung der Schmutzentfernung und -lösung, ein leichtes Abspülen und - je nach Wunsch - auch für Schaumregulierung. Damit ist die Grundlage für die Schmutzentfernung lipidhaltiger Verschmutzungen gegeben.

Emulgatoren ermöglichen oder erleichtern die gleichmäßige Verteilung zweier oder mehrerer miteinander nicht mischbarer Phasen und verhindern gleichzeitig deren Entmischung.

Ende der vierziger Jahre wurde ein System entwickelt, das die Auswahl von Emulgatoren erleichtern sollte. Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen öllösliche, hydrophobe Emulgatoren, Zahlen über 11 wasserlösliche, hydrophile. Der HLB-Wert sagt etwas über das Gleichgewicht der Größe und Stärke der hydrophilen und der lipophilen Gruppen eines Emulgators aus. Aus diesen Überlegungen lässt sich ableiten, dass auch die Wirksamkeit eines Emulgators durch seinen HLB-Wert charakterisiert werden kann. Die folgende Aufstellung zeigt den Zusammenhang zwischen HLB-Wert und möglichem Anwendungsgebiet:

| | |
|---|---|
| **HLB-Wert** | **Anwendungsgebiet** |
| 0 bis 3 | Entschäumer |
| 3 bis 8 | W/O-Ernulgator |
| 7 bis 9 | Netzmittel |
| 8 bis 18 | O/W-Emulgator |
| 12 bis 18 | Lösungsvermittler |

Der HLB-Wert eines Emulgators lässt sich auch aus Inkrementen zusammensetzen, wobei die HLB-Inkremente für die verschiedenen hydrophilen und hydrophoben Gruppen, aus denen sich ein Molekül zusammensetzt, Tabellenwerken entnommen werden können. Auf diese Weise lassen sich auch für waschaktive Tenside HLB-Werte ermitteln, obwohl das HLB-System ursprünglich nur für Emulgatoren konzipiert worden ist. Es zeigt sich, dass waschaktive Substanzen in der Regel HLB-Werte aufweisen, die deutlich größer als 20 sind.

Ein Problem bei der Anwendung von Reinigungsprodukten besteht in dem Umstand, dass diese meist tensidhaltigen Zubereitungen die Haut stark entfetten und austrocknen. Um diesem Missstand abzuhelfen, können den Zubereitungen entweder Öle zugesetzt (sogenannte Duschöle) oder die Haut nach dem Waschen mit einer Pflegeemulsion behandelt werden. Meist jedoch lässt die rückfettende Wirkung herkömmlicher Duschöle zu wünschen übrig, da der größte Teil des Produktes nach dem Auftragen auf die Haut wieder abgespült wird. Auch können in derartige Zubereitungen nur unzureichend wasserlösliche Pflegesstoffe eingearbeitet werden. Das Eincremen der Haut mit Pflegeemulsionen nach dem Waschvorgang ist zwar äußerst effektiv, jedoch vergrößert sich der Zeitaufwand für die Körperpflege deutlich, da die beiden kosmetischen Einzelschritte der Reinigung und Pflege nacheinander erfolgen. So hat es nicht an Versuchen gemangelt, tensidhaltige Reinigungszubereitungen und Pflegeemulsionen miteinander zu kombinieren und tensidhaltige Pflegeemulsionen zur Körperteinigung zu entwickeln.

Der Nachteil am Stande der Technik bei derartigen Reinigungsemulsionen besteht jedoch in Ihrem geringen Schäumvermögen. Dies führt zu einer deutlich reduzierten Reinigungsleistung. Die Ursache für dieses Phänomen liegt darin begründet, dass ein Großteil der Tenside an den fein verteilten Öltröpfchen der Emulsion gebunden ist. Dieses Problem lässt sich aus dermatologischen wie aus formulierungstechnischen Gründen nicht ohne weiteres durch einen höheren Tensidanteil in der Zubereitung kompensieren. Da Emulsionen durch die Zugabe von Tensiden im allgemeinen zerstört werden, ist darüber hinaus die Wahl des Tensidsystems stark eingeschränkt, und den erhaltenen Reinigungszubereitungen liegen teure und komplizierte Rezepturen zugrunde.

Ein weiterer Lösungsansatz für dieses Problem bestand daher darin, die tensidhaltige Reinigungszubereitung und die Pflegeemulsion in zwei getrennten Kammern eines gemeinsamen Verpackungssystems aufzubewahren und aus diesem heraus anzuwenden. Dieser Weg wurde beispielsweise in der WO 01/70923, der WO 96/02230, der WO 96/37420 und der WO 98/33477 beschritten. Nachteilig am Stande der Technik ist jedoch der Umstand, dass sich die beiden Einzelkomponenten (tensidhaltige Reinigungszubereitung und Pflegeemulsion) in diesen Produkten nur schlecht miteinander vermischen lassen.

Es war daher die Aufgabe der vorliegenden Erfindung, die Mängel des Standes der Technik zu beseitigen oder zumindest zu lindern und ein pflegendes Kosmetikum zur Reinigung der Haut und Hautanhangsgebilde zu entwickeln.

Überraschend gelöst wird die Aufgabe durch ein Kosmetikum und/oder Dermatikums enthaltend
a) eine kosmetische und/oder dermatologische Reinigungszubereitung A,
b) eine Zubereitung B, die in Form einer Öl-in-Wasser-Emulsion (O/W-Emulsion) vorliegt, die mit einem oder mehreren Stearat-Emulgatoren emulgiert ist, dadurch gekennzeichnet, dass die beiden Zubereitungen A und B in getrennten Kammern eines gemeinsamen Verpackungsbehältnisses aufbewahrt, im Gewichtsverhältnis von 10:1 bis 1:10 vorliegen, wobei beide Zubereitungen dem Verpackungsbehältnis entweder durch eine gemeinsame Öffnung gleichzeitig entnommen werden und beide Zubereitungen entweder vor dem Austritt aus der Entnahmeöffnung vermischt oder in Form einer streifenförmigen Gesamtzubereitung aus der Entnahmeöffnung austreten, oder zwei getrennten Öffnungen entnommen werden.

Erfindungsgemäß vorteilhaft enthält das erfindungsgemäße Kosmetikum und/oder Dermatikum in der Zubereitung B ein oder mehrere Stearat-Emulgatoren in einer Gesamtkonzentration von 0,5 bis 12 Gewichts-%, bevorzugt in einer Konzentration von 1,5 bis 8 Gewichts-% und ganz besonders bevorzugt in einer Konzentration von 2 bis 4 Gewichts-%, jeweils bezogen auf das Gesamtgewicht der Zubereitung B.

Die erfindungsgemäßen Kosmetika bzw. Dermatika zeigen ungewöhnlich gute Resultate hinsichtlich der Schaumcharakteristik und der Mischbarkeit der O/W-Emulsion mit der Reinigungszubereitung. Die erfindungsgemäßen Zubereitungen hinterlassen nach ihrer Anwendung ein sauberes, geschmeidiges und gepflegtes Gefühl auf der Haut zurück. Dies war insbesondere deshalb überraschend, da in der WO 01/70923 von Anwendungstests mit einer Kombination aus einer O/W-Emulsion und einer Reinigungszubereitung an Schweinehäuten berichtet wird, die eine besondere Pflegewirkung von derartigen Produkten unwahrscheinlich erscheinen ließen

Der oder die O/W-Emulgatoren auf Stearat-Basis werden erfindungsgemäß vorzugsweise aus der folgenden Gruppe gewählt
Glycerylstearat in Kombination mit Ceteareth-20, Ceteareth-25, Ceteareth-6 in Kombination mit Stearylalkohol, Cetylstearylalkohol in Kombination mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl, PEG-6 Capryl-säure/Caprinsäureglyceride, Glyceryloleat in Kombination mit PEG-9-Stearat, PEG-20 Stearat, PEG-30-Stearat, PEG-40-Stearat, PEG-100-Stearat, Glycerylstearat in Kombination mit PEG-100 Stearat, Glycerylmyristat, Glyceryllaurat, PEG-40-Sorbitanperoleat, Laureth-4, Ceteareth-3, Isostearylglycerylether, Cetylstearylalkohol in Kombination mit Natrium cetylstearylsulfat, Laureth-23, Steareth-2, Glycerylstearat in Kombination mit PEG-30 Stearat, PEG-40-Stearat, Glycol Distearat, PEG-22-Dodecyl Glycol Copolymer, Polyglyceryl-2-PEG-4-Stearat, Ceteareth-12, Ceteareth-20, Ceteareth-30, Methyl-glucosesesquistearat, Steareth-10, PEG-20-Stearat, Steareth-2 in Kombination mit PEG-8 Distearat, Steareth-21, Steareth-20, Isosteareth-20, PEG-45/Dodecylglycol-Copolymer, Methoxy-PEG-22/Dodecylglycol-Copolymer, PEG-40-Sorbitanperoleat, PEG-40-Sorbitanperisostearat, PEG-20-Glycerylstearat, PEG-20-Glycerylstearat, PEG-8-Bienenwachs, Polyglyceryl-2-laurat, Isostearyldiglycerylsuccinat,Stearamidopropyl-PG-dimoniumchloridphosphat, Glycerylstearat SE, Ceteth-20, Triethylcitrat, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglycerylmethylglucosedistearat, Kaliumcetylphosphat, Isosteareth-10, Polyglyceryl-2-sesquiisostearat, Ceteth-10, Oleth-20, lsoceteth-20, Glycerylstearat in Kombination mit Ceteareth-20, Ceteareth-12, Cetylstearylalkohol und Cetylpalmitat, Cetylstearylalkohol in Kombination mit PEG-20 Stearat, PEG-30-Stearat, PEG-40-Stearat, PEG-100-Stearat.

Erfindungsgemäß bevorzugte Stearat-Emulgatoren sind Glycerylstearatcitrat, Glycerylstearat, PEG-40-Stearat, Triglycerinmethylglucosestearat, Natriumstearat, Kaliumstearat, TEA-Stearat, Sorbitanstearat, Stearylalkohol, Polyethylenglycol(21)stearylether und/oder Polyethylenglycol(2)-stearylether. Erfindungsgemäß besonders bevorzugt ist Glycerylstearat SE (=Glycerinmonostearat, selbstemulgierend) als Emulgator.

Es ist erfindungsgemäß vorteilhaft, wenn als Zubereitung A eine tensidhaltige Reinigungszubereitung eingesetzt wird.

Die erfindungsgemäße Zubereitung A kann vorteilhafter Weise ein oder mehrere Tenside enthalten. Erfindungsgemäß vorteilhaft können sowohl anionische, kationische, nichtionische und zwitterionische Tenside eingesetzt werden. Erfindungsgemäß bevorzugt ist insbesondere der Einsatz von einem oder mehreren anionischen Tensiden in der Zubereitung A.

Vorteilhafte waschaktive anionische Tenside im Sinne der vorliegenden Erfindung sind Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie
■ Acylisethionate, z.B. Natrium-/ Ammoniumcocoylisethionat,
■ Sulfosuccinate, beispielsweise Dioctylnatriumsulfosuccinat,Dinatriumlaurethsulfosuccinat, Dinatriumlaurylsulfosuccinat und Dinatriumundecylenamido MEA-Sulfosüccinat, Dinatrium PEG-5 Laurylcitratsulfosuccinat und Derivate,
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat
■ Alkylsulfate, beispielsweise Natrium-, Ammonium- und TEA- Laurylsulfat.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.

Vorteilhafte waschaktive amphotere Tenside im Sinne der vorliegenden Erfindung sind
■ Acyl-/dialkylethylendiamine, beispielsweise Natriumacylamphoacetat, Dinatriumacylamphodipropionat, Dinatriumalkylamphodiacetat,Natriumacylamphohydroxypropylsulfonat, Dinatriumacylamphodiacetat und Natriumacylamphopropioilat,

Vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, wie beispielsweise DEA-Oleth-10 Phosphat und Dilaureth-4 Phosphat,
Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat und N-Kokosfettsäureamidoethyl-N-hydroxyethylglycinat Natriumsalze und deren Derivate

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole,
■ ethoxylierte Amine
insbesondere deren Salze.

Weitere geeignete nicht-ionische Tenside im Sinne der vorliegenden Erfindung sind ferner Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß besonders vorteilhaft, wenn als Tenside in der Zubereitung A Natriumlaurethsulfat, Natriummyrethsulfat, Cocamidopropylbetain, Alkylpolylglucosid, Natriumcocoylglutamat und/oder Natriumcocoamphoacetat eingesetzt werden.

Erfindungsgemäß bevorzugt sind Tensidkombinationen aus Alkylethersulfaten mit amphoteren Cotensiden, wobei Tensidkombinationen aus Natriumlaurethsulfat und Cocamidopropylbetain oder Natriumlaurethsulfat, Cocamidopropylbetain und Natriumcocoylglutamat einerseits sowie Tensidkombinationen aus Natriummyrethsulfat und Decylglycosid andererseits besonders bevorzugt ist. Insbesondere mit diesen Tensidkombinationen lassen sich Reinigungszubereitungen mit hoher Schaumleistung, sowie besonders cremigem Schaum bei der Anwendung herstellen.

Es ist vorteilhaft im Sinn der vorliegenden Erfindung, wenn der Gehalt an einem oder mehreren waschaktiven Tensiden in der kosmetischen Zubereitung A aus dem Bereich von 1 bis 30 Gewichts-%, bevorzugt von 5 bis 25 Gew.-%, ganz besonders vorteilhaft von 10 bis 20 Gew.-% gewählt wird, jeweils bezogen auf das Gesamtgewicht der Zubereitung A.

Erfindungsgemäß vorteilhaft kann mindestens eine der erfindungsgemäßen Zubereitungen Polysorbate enthalten

Im Sinne der Erfindung vorteilhafte Polysorbate sind beispielsweise das
- Polyoxyethylen(20)sorbitanmonolaurat (Tween 20, CAS-Nr.9005-64-5)
- Polyoxyethylen(4)sorbitanmonolaurat (Tween 21, CAS-Nr.9005-64-5)
- Polyoxyethylen(4)sorbitanmonostearat (Tween 61, CAS-Nr. 9005-67-8)
- Polyoxyethylen(20)sorbitantristearat (Tween 65, CAS-Nr. 9005-71-4)
- Polyoxyethylen(20)sorbitanmonooleat (Tween 80, CAS-Nr. 9005-65-6)
- Polyoxyethylen(5)sorbitanmonooleat (Tween 81, CAS-Nr. 9005-65-5)
- Polyoxyethylen(20)sorbitantrioleat (Tween 85, CAS-Nr. 9005-70-3).

Diese werden erfindungsgemäß vorteilhaft in einer Konzentration von 0,1 bis 5 Gewichts-% und insbesondere in einer Konzentration von 1,5 bis 2,5 Gewichts-%, bezogen auf das Gesamtgewicht der jeweiligen Zubereitung einzeln oder als Mischung mehrer Polysorbate, eingesetzt.

Die erfindungsgemäßen Zubereitungen A und B können als wässrige Lösung oder wässrige Phase einer Emulsion neben Wasser erfindungsgemäß auch andere Inhaltsstöffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder monoethylether und analoge Produkte, ferner Alkohole niedriger C-Zahl, z.B. Ethanol, Isopropanol, 1,2-Propandiol und Glycerin.

Auch die Zubereitung A kann zusätzlich neben einer oder mehreren Wasserphasen eine oder mehrere Ölphasen enthalten und beispielsweise in Form von W/O-, O/W-, W/O/W- oder O/W/O-Emulsionen vorliegen. Solche Formulierungen können vorzugsweise auch eine Mikroemulsion (z. B. eine PIT-Emulsion), eine Feststoff-Emulsionen (d. h. eine Emulsion, welche durch Feststoffe stabilisiert ist, z. B. eine Pickering-Emulsion) sein, wobei transparente oder transluzente Mikroemulsionen erfindungsgemäß besonders bevorzugt sind.

Es ist erfindungsgemäß bevorzugt, wenn die Zubereitung A eine wässrige Zubereitung oder ein zu einem Gel-verdickte wässrige Zubereitung darstellt.

Die erfindungsgemäßen Zubereitungen können vorteilhaft anfeuchtende bzw. feuchthaltende Mittel (sogenannte Moisturizer) enthalten. Vorteilhafte feuchthaltende Mittel im Sinne der vorliegenden Erfindung sind beispielsweise Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil; polymere Moisturizer aus der Gruppe der wasserlöslichen und/oder in Wasser quellbaren, und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der jeweiligen Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der jeweiligen Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der jeweiligen Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen könnten.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

In die erfindungsgemäßen Zubereitungen können aber auch andere pharmazeutisch oder dermatologisch wirkende Substanzen wie beispielsweise die Haut beruhigende und pflegende Substanzen eingearbeitet sein. Hierzu zählen beispielsweise Panthenol, Allantoin, Tannin, Antihistaminika, Antiphlogistika, Glucocorticoide (z.B. Hydrocortison) sowie Pflanzenwirkstoffe wie Azulen und Bisabolol, Glycyrrhizin, Hamamelin und Pflanzenextrakte wie Kamille, aloe vera, Hamazelis, Süßholzwurzel. Auch die Vitamin D₃₋analoga Tacalcitol, Calcipotriol, Tacalcitol, Colecalciferol sowie Calcitrol (Vitamin D₃) und/oder Fumarsäureester können erfolgreich in die Zubereitungen hineinformuliert werden.

Die Ölphase bzw. Ölphasen der erfindungsgemäßen Formulierungen wird/werden vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegendenEreindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann eine Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Cocoglyceride (z. B. Myritol® 331 von Henkel), C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Halistar AB*) und/oderDiethylhexylnaphthalat (*Corapan®TQ von Haarmenn & Reimer*)*.*

Auch beliebige Abmischungen solcher Öl- und Wachskomponenten sind vorteilhaft im Sinne der vorliegenden Erfindung einzusetzen.

Ferner kann eine Ölphase ebenfalls vorteilhaft auch unpolare Öle enthalten beispielsweise solche, welche gewählt werden aus der Gruppe der verzweigten und unverzweigten Kohlenwasserstoffe und- -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann eine Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, werden auch als Polydimethylsiloxan bzw. Dimethicon (INCI) bezeichnet. Dimethicone gibt es in verschiedenen Kettenlängen bzw. mit verschiedenen Molekulargewichten

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCl: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die erfindungsgemäßen Zubereitungen können auch alle nach der Kosmetikverordnung zugelassenen wasserlöslichen und/oder öllöslichen UV-A-, UV-B- und/oder Breitbandfiltersubstanzen enthalten.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, weitere Pflanzenextrakte, Vitamine, Wirkstoffe, Konservierungsmittel, Bakterizide, Repellentien, Selbstbräuner, Depigmentierungsmittel, Pigmente, die eine färbende Wirkung haben, weichmachende, anfeuchtende und/oder feuchthaltende Substanzen, oder andere übliche Bestandteile einer kosmetischen oder dermatologischen Formulierung wie Emulgatoren, Polymere, Schaumstabilisatoren und Elektrolyte.

Die erfindungsgemäße Zubereitung kann erfindungsgemäß vorteilhaft ein oder mehrere Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Nätriumnitrat |
| E 220 | Schwefeldioxid | E 252 | Kaliumnitrat |
| E 221 | Natriumsulfit- | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropioriat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid Benzalkoniümchlorid, Benzylalkohol.
Es ist dabei erfindungsgemäß besonders bevorzugt, wenn als Konservierungsstoffe Benzoesäure und/oder Salicylsäure und/oder deren Derivate oder Salze eingesetzt werden.

Erfindungsgemäß vorteilhaft sind ein oder mehrere Konservierungsstoffe in einer Konzentration von 2 Gewichts-% oder kleiner 2 Gewichts-%, bevorzugt 1,5 Gewichts-% oder kleiner 1,5 Gewichts-% und besonders bevorzugt 1 Gewichts-% oder kleiner 1 Gewichts-%, jeweils bezogen auf das Gesamtgewicht der Zubereitung A oder B in einer der beiden Teilzubereitungen oder in beiden Teilzubereitungen enthalten.

Die erfindungsgemäßen Zubereitungen enthalten vorteilhafter Weise einen oder mehrere Konditionierer. Erfindungsgemäß bevorzugte Konditionierer sind beispielsweise alle Verbindungen, welche im *International Cosmetic Ingredient Dictionary and Handbook* (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Ein Teil dieser Verbindungen wird unter den Bestandteilen der wässrigen Phase und der Ölphase namentlich aufgeführt. Weitere erfindungsgemäß vorteilhafte Konditionierer stellen beispielsweise die nach der internationalen Nomenklatur für kosmetische Inhaltsstoffe (INCl) als Polyquaternium benannten Verbindungen dar (insbesondere Polyquaternium-1 bis Polyquaternium-56).

Es ist erfindungsgemäß vorteilhaft, mindestens einer der erfindungsgemäßen Zubereitungen Glittersoffe und/oder andere Effektstoffe zuzusetzen.

Auch ist es vorteilhaft im Sinne der vorliegenden Erfindung, wenn mindestens eine der beiden Zubereitungen A und B ein oder mehrere Hydrokolloide in einer Konzentration von 0,01 bis 10 Gewichts-%, bevorzugt in einer Konzentration von 0,1 bis 7 Gewichts-% und ganz besonders bevorzugt in einer Konzentration von 0,3 bis 5 Gewichts-%, jeweils bezogen auf das Gesamtgewicht der Zubereitung enthält.

Als erfindungsgemäß vorteilhafte Hydrokolloide werden Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Celluloseether, Hydroxyethyl-und -propylcellulosederivate, Polysaccharide, Polyacryl- und Polymethacryl-Verbindungen, Vinylpolymere, Polycarbonsäuren, Polyether, Polyimine, Polyamide, Polykieselsäuren, Tonmineralien, Zeolithe, Kieselsäuren eingesetzt

Erfindungsgemäß bevorzugte Hydrokolloide sind beispielsweise Methylcellulosen, als welche die Methylether der Cellulose bezeichnet werden. Sie zeichnen sich durch die folgende Strukturformel aus 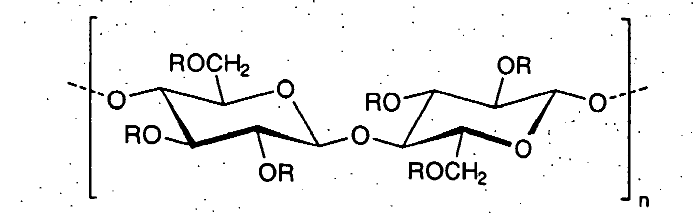 in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder CH2-COONa darstellen kann. Besonders bevorzugt ist,die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Spezies unter aeroben Bedingungen mit einem Molekulargewicht von 2x106 bis 24x106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Spezies unter aeroben Bedingungen mit einem Molekulargewicht von 2-15 106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlant., zu den Florideen zählenden Rotalgen (Chondrus crispus u. Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht löslich ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt . Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-a-D-galactose-2-sulfat in 1,4-Bindung aufgebaute τ-Carrageenan ist sowohl wasserlöslich als auch gelbildend. Weitere Carrageen-Typen werden ebenfalls mit griechischen Buchstaben bezeichnet: α, β, γ, µ ν, ξ, π, ω, χ. Auch die Art vorhandener Kationen (K+, NH4+, Na+, Mg2+, Ca2+) beeinflusst die Löslichkeit der Carrageene

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur aus:

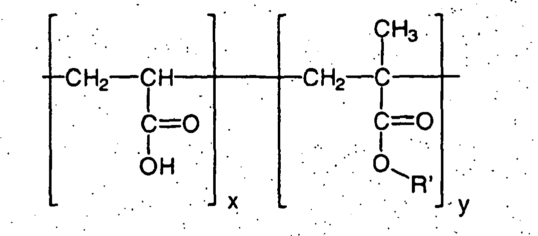

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkyl-acrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Ferner vorteilhaft sind Copolymere aus C10-30-Alkylacrylate und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCl-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 bei der NOVEON Inc. erhältlichen.

Vorteilhaft sind ferner Verbindungen, die die INCl-Bezeichnung acrylates/C12-24 pareth-25 acrylate copolymer" (unter der Handelsbezeichnungen Synthalen® W2000 bei der 3V Inc. erhältlich), die die INCI-Bezeichnung "acrylates/steareth-20 methacrylate copolymer" (unter der Handelsbezeichnungen Aculyn® 22 bei der International Specialty Product Corp. erhältlich), die die INCI-Bezeichnung "acrylates/steareth-20 itaconate copolymer" (unter der Handelsbezeichnungen Structure 2001® bei der National Starch erhältlich), die die INCI-Bezeichnung "acrylates/aminoacrylates/C10-30 alkyl PEG-20 itaconate copolymer" (unter der Handelsbezeichnungen Structure Plus® bei der National Starch erhältlich) und ähnliche Polymere.

Erfindungsgemäß bevorzugt ist es insbesondere, neutralisierte oder teilneutralisierte Polyacrylate (z.B. Carbopole der Firma Noveon) einzusetzen.

Die Gesamtmenge an einem oder mehreren Hydrokolloiden wird vorteilhaft kleiner als 5 Gew.-%, bevorzugt zwischen 0,1 und 5 Gew.-%, bezogen auf das Gesamtgewicht der jeweiligen Zubereitung, gewählt.

Es ist vorteilhaft im Sinn der vorliegenden Erfindung, wenn der Gehalt an einem oder mehreren Polyacrylaten in einer kosmetischen oder dermatologischen Reinigungsemulsion aus dem Bereich von 0,1 bis 8 Gew.-%, ganz besonders vorteilhaft von 0,1 bis 5 Gew.-% gewählt wird, jeweils bezogen auf das Gesamtgewicht der jeweiligen Zubereitung.

Erfindungsgemäße Verpackungsbehältnisse können vorteilhaft aus einer Kunststoffflasche oder einer Aerosoldose bestehen. Erfindungsgemäß vorteilhaft kann auch mindestens eine der beiden Zubereitungen mit einem Treibmittel aufgeschäumt werden.

Als erfindungsgemäß vorteilhafte Verpackungsbehältnisse können beispielsweise Verpackungen gewählt werden, wie sie in der WO 96/37420 oder der GB 2 180 215 offenbart sind. Auch Verpackungssysteme wie sie von mehrfarbig gestreifter Zahnpasta her bekannt sind, lassen sich erfindungsgemäß vorteilhaft verwenden unter der Maßgabe, dass die beiden Zubereitungen durch eine undurchlässige Sperre voneinander getrennt sind und erst beim Austritt aus der Verpackung zu der bekannten streifenförmigen Gesamtzusammensetzung zusammengesetzt werden.

Vorteilhafte Verpackungsbehältnisse stellen ferner mit zwei Kammern versehene Quetschflaschen dar, die entweder für beide Zubereitungen eine gemeinsame Entnahmeöffnung oder aber zwei separate Entnahmeöffnungen aufweisen.

Erfindungsgemäß vorteilhafte Verpackungsbehältnisse stellen auch Doppelkammertuben mit ein oder zwei Entnahmeöffungen dar.

Nicht zuletzt ist es erfindungsgemäß vorteilhaft, wenn beide Zubereitungen zusammen oder getrennt mit Hilfe eines Pump-und/oder Dosierspenders dem Verpackungsbehältnis entnommen werden können.

Generell bevorzugt sind Verpackungsbehältnisse, bei denen die beiden erfindungsgemäßen Zubereitungen A und B dem Behältnis aus zwei unterschiedlichen Entnahmeöffnungen entnommen werden.

Es ist erfindungsgemäß vorteilhaft, wenn die beiden Zubereitungen A und B unterschiedlich gefärbt und/oder mit unterschiedlichen Effektstoffen beladen sind.

Erfindungsgemäß ist die Verwendung des erfindungsgemäßen Kosmetikums und/oder Dermatikums zur Reinigung der Haut und/oder Hautanhangsgebilde.

Erfindungsgemäß ist die Verwendung des erfindungsgemäßen Kosmetikums und/oder Dermatikums zur Pflege der Haut und/oder Hautanhangsgebilde.

Erfindungsgemäß ist die Verwendung des erfindungsgemäßen Kosmetikums und/oder Dermatikums als Duschgel und/oder Wannenbad.

Ferner eignen sich die erfindungsgemäßen Produkte hervorragend zur Reinigung und Pflege von Gegenständen des täglichen Lebens (z.B. Geschirr, Tisch- und Schrankflächen, Autos).

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

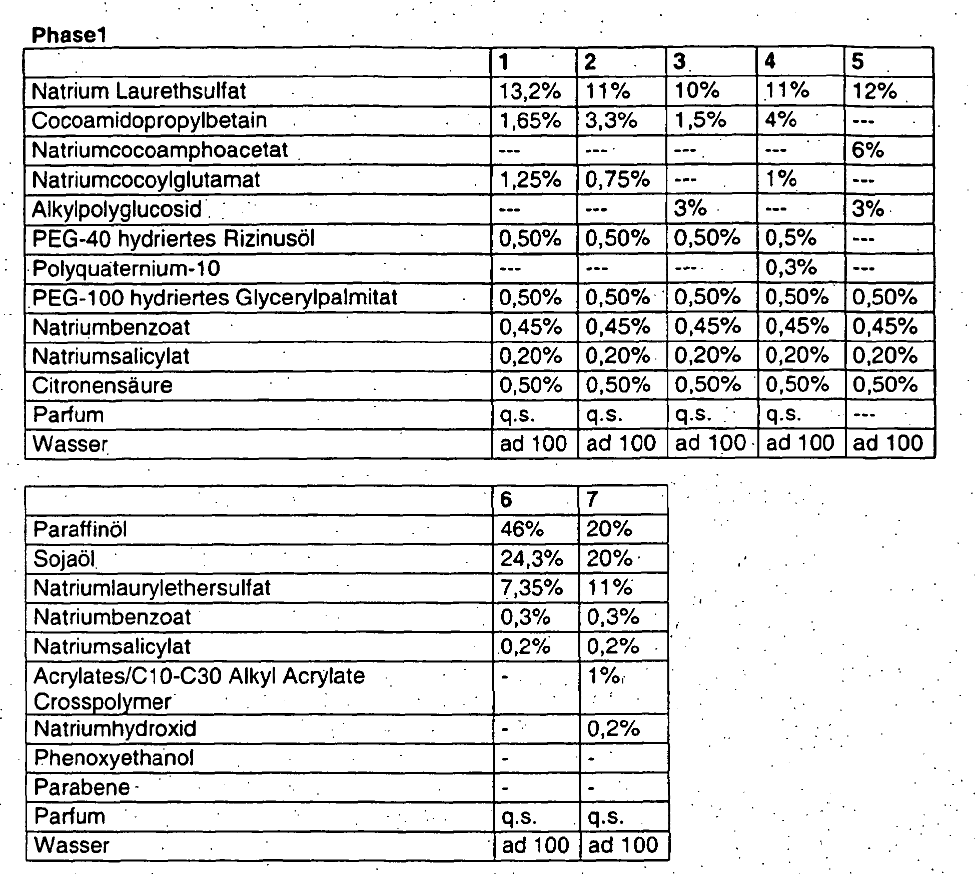

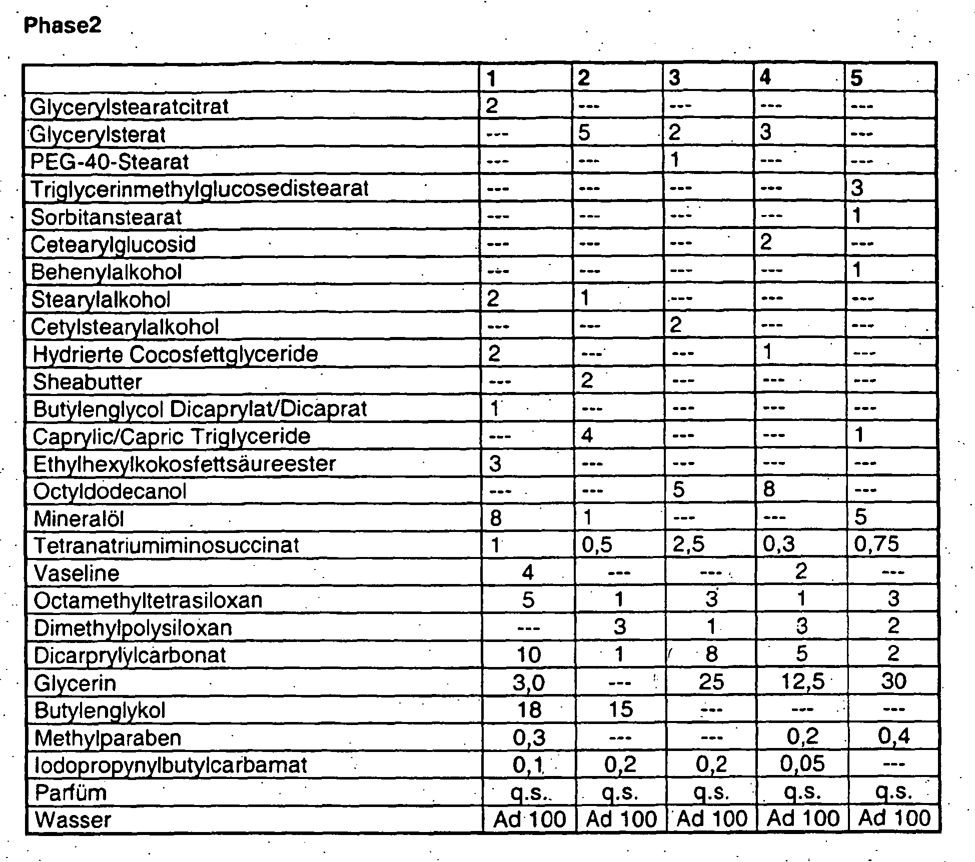

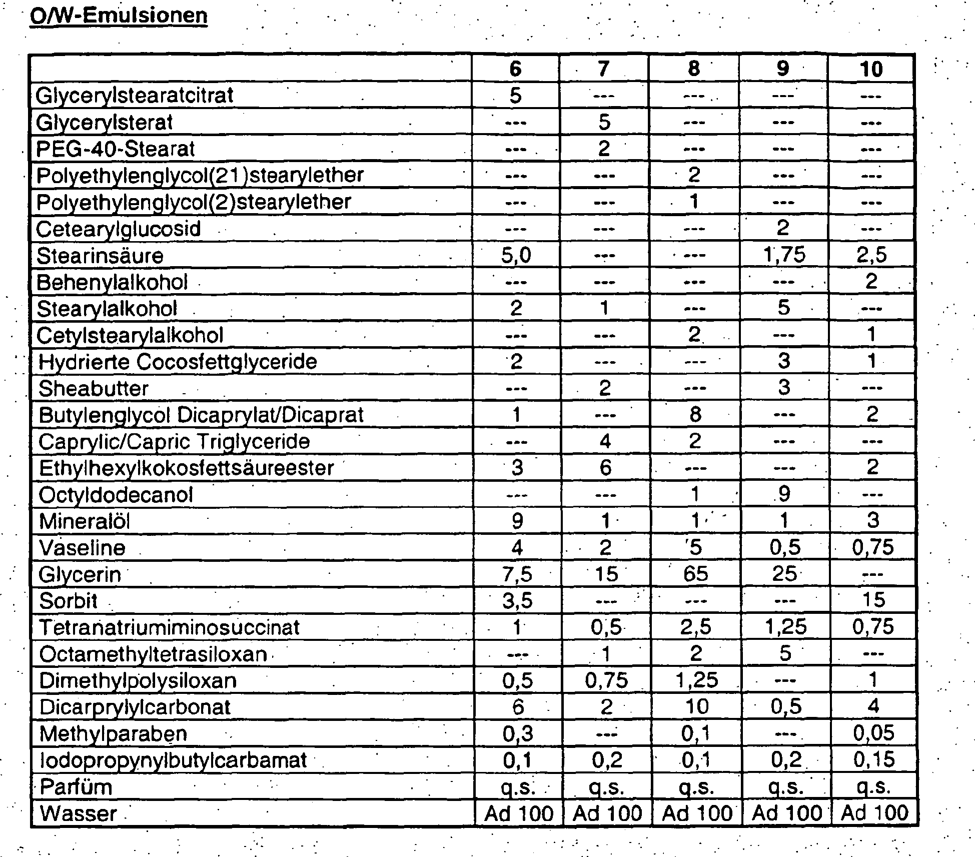

## Patentansprüche

1. Kosmetikum und/oder Dermatikum enthaltend
a) eine kosmetische und/oder dermatologische Reinigungszubereitung A,
b) eine Zubereitung B, die in Form einer Öl-in-Wasser-Emulsion (O/W-Emulsion) vorliegt, die mit einem oder mehreren Stearat-Emulgatoren emulgiert ist,
**dadurch gekennzeichnet, dass** die beiden Zubereitungen A und B in getrennten Kammern eines gemeinsamen Verpackungsbehältnisses aufbewahrt, im Gewichtsverhältnis von 10:1 bis 1:10 vorliegen, wobei beide Zubereitungen dem Verpackungsbehältnis entweder durch eine gemeinsame Öffnung gleichzeitig entnommen werden und beide Zubereitungen entweder vor dem Austritt aus der Entnahmeöffnung vermischt oder in Form einer streifenförmigen Gesamtzubereitung aus der Entnahmeöffnung austreten, oder zwei getrennten Öffnungen entnommen werden.

2. Kosmetikum und/oder Dermatikum nach Anspruch 1, **dadurch gekennzeichnet, dass** in Zubereitung B ein oder mehrere Stearat-Emulgatoren in einer Gesamtkonzentration von 0,5 bis 12 Gewichts-%, bezogen auf das Gesamtgewicht der Zubereitung B, eingesetzt werden.

3. Kosmetikum und/oder Dermatikum nach einem der Ansprüche 1 oder 2, **dadurch gekennzeichnet, dass** in der Zubereitung B als Stearat-Ermulgatoren Glycerylstearatcitrat, Glycerylstearat, PEG-40-Stearat, Triglycerinmethylglucosestearat, Sorbitanstearat, Polyethylenglycol(21)stearylether und/oder Polyethylenglycol(2)-stearylether eingesetzt werden.

4. Kosmetikum und/oder Dermatikum nach einem der Ansprüche 1 bis 3, **dadurch gekennzeichnet, dass** Zubereitung A anionische, kationische, amphotere und/oder nichtionische Tenside in einer Gesamtkonzentration von 1 bis 30 Gewichts-%, bezogen auf das Gesamtgewicht der Zubereitung A, enthält.

5. Kosmetikum und/oder Dermatikum nach einem der Ansprüche 1 bis 4, **dadurch gekennzeichnet, dass** in der Zubereitung A Natriumlaurethsulfat, Natriummyrethsulfat, Cocamidopropylbetain, Alkylpolyglucosid, Natriumcocoylglutamat und/oder Natriumcocoamphoacetat eingesetzt werden.

6. Kosmetikum und/oder Dermatikum nach einem der Ansprüche 1 bis 5, **dadurch gekennzeichnet, dass** Verpackungsbehältnisse verwendet werden, bei denen die beiden erfindungsgemäßen Zubereitungen A und B dem Behältnis aus zwei unterschiedlichen Entnahmeöffnungen entnommen werden

7. Verwendung eines Kosmetikums und/oder Dermatikums nach einem der vorhergehenden Ansprüche zur Reinigung der Haut und/oder Hautanhangsgebilde.

8. Verwendung eines Kosmetikums und/oder Dermatikums nach einem der vorhergehenden Ansprüche als Duschgel und/oder Wannenbad.

## Claims

1. Cosmetic composition and/or dermatological composition comprising
a) a cosmetic and/or dermatological cleaning preparation A,
b) a preparation B, which is in the form of an oil-in-water emulsion (O/W emulsion), which is emulsified with one or more stearate emulsifiers,
**characterized in that** the two preparations A and B are stored in separate chambers of a common packaging container, are present in the weight ratio of from 10:1 to 1:10, where both preparations are removed from the packaging container either through a common opening at the same time and both preparations are mixed either prior to emerging from the discharge opening or emerge in the form of a striped overall preparation from the discharge opening, or are removed from two separate openings.

2. Cosmetic composition and/or dermatological composition according to Claim 1, **characterized in that**, in preparation B, one or more stearate emulsifiers are used in an overall concentration of from 0.5 to 12% by weight, based on the total weight of preparation B.

3. Cosmetic composition and/or dermatological composition according to one of Claims 1 or 2, **characterized in that**, in preparation B, the stearate emulsifiers used are glyceryl stearate citrate, glyceryl stearate, PEG-40 stearate, triglyceryl methylglucose stearate, sorbitan stearate, polyethylene glycol(21) stearyl ether and/or polyethylene glycol(2) stearyl ether.

4. Cosmetic composition and/or dermatological composition according to one of Claims 1 to 3, **characterized in that** preparation A comprises anionic, cationic, amphoteric and/or nonionic surfactants in a total concentration of from 1 to 30% by weight, based on the total weight of preparation A.

5. Cosmetic composition and/or dermatological composition according to one of Claims 1 to 4, **characterized in that**, in preparation A, sodium laureth sulphate, sodium myreth sulphate, cocamidopropylbetaine, alkyl polyglucoside, sodium cocoyl glutamate and/or sodium cocoamphoacetate are used.

6. Cosmetic composition and/or dermatological composition according to one of Claims 1 to 5, **characterized in that** packaging containers are used in which the two preparations A and B according to the invention are removed from two different discharge openings.

7. Use of a cosmetic composition and/or dermatological composition according to one of the preceding claims for cleaning the skin and/or skin appendages.

8. Use of a cosmetic composition and/or dermatological composition according to one of the preceding claims as shower gel and/or bath.

## Revendications

1. Produit cosmétique et/ou dermatologique contenant
a) une préparation cosmétique et/ou dermatologique de nettoyage A,
b) une préparation B se trouvant sous forme d'une émulsion huile-dans-eau (émulsion H/E) qui est émulsionnée avec un ou plusieurs émulsifiants stéarate,
**caractérisé en ce que** les deux préparations A et B sont conservées en un rapport de 10:1 à 1:10 dans des compartiments séparés d'un contenant d'emballage commun, soit les deux préparations étant prélevées simultanément du contenant d'emballage par un orifice commun, et les deux préparations soit étant mélangées avant la sortie de l'orifice de prélèvement, soit sortant de l'orifice de prélèvement sous forme d'une préparation totale en bande, soit étant prélevées de deux orifices distincts.

2. Produit cosmétique et/ou dermatologique selon la revendication 1, **caractérisé en ce que** dans la préparation B sont utilisés un ou plusieurs émulsifiants stéarate à une concentration totale de 0,5 à 12 % en poids, par rapport au poids total de la préparation B.

3. Produit cosmétique et/ou dermatologique selon la revendication 1 ou 2, **caractérisé en ce que** comme émulsifiants stéarate dans la préparation B on utilise le citrate-stéarate de glycéryle, le stéarate de glycéryle, le stéarate de PEG-40, le stéarate de triglycérol-méthylglucose, le stéarate de sorbitanne, l'éther stéarylique de polyéthylèneglycol-(21) et/ou l'éther stéarylique de polyéthylèneglycol- (2) .

4. Produit cosmétique et/ou dermatologique selon l'une quelconque des revendications 1 à 3, **caractérisé en ce que** la préparation A contient des tensioactifs anioniques, cationiques, amphotères et/ou non ioniques à une concentration totale de 1 à 30 % en poids, par rapport au poids total de la préparation A.

5. Produit cosmétique et/ou dermatologique selon l'une quelconque des revendications 1 à 4, **caractérisé en ce que** dans la préparation A on utilise du laurethsulfate de sodium, du myrethsulfate de sodium, de la cocosamidopropylbétaïne, un alkylpolyglucoside, du cocoylglutamate de sodium et/ou du cocoamphoacétate de sodium.

6. Produit cosmétique et/ou dermatologique selon l'une quelconque des revendications 1 à 5,
**caractérisé en ce qu'**on utilise des contenants d'emballage dans lesquels les deux préparations A et B selon l'invention sont prélevées du contenant par deux orifices de prélèvement différents.

7. Utilisation d'un produit cosmétique et/ou dermatologique selon l'une quelconque des revendications précédentes, pour le nettoyage de la peau et/ou d'annexes de la peau.

8. Utilisation d'un produit cosmétique et/ou dermatologique selon l'une quelconque des revendications précédentes, en tant que gel de douche et/ou gel de bain.
